# EUROPEAN PATENT APPLICATION

(11) **EP 4 809 906 A1**
(43) Date of publication of application: **23.09.2026**
(21) Application number: 25165062.8
(22) Date of filing: 20.03.2025
(51) Int. Cl.: A61B 5/00, A61B 5/0275

(54) **METHOD FOR PROVIDING A PERFUSION BASED COMPLICATION PREDICTION AND CLINICAL DECISION SUPPORT SYSTEM**

(71) Applicant: Quest Photonic Devices B.V., 1771 SR Wieringerwerf (NL)
(72) Inventor: TIEBIE, Kasper, 1761 AJ Anna Paulowna (NL); BUCHINI LABAYEN, Ana, 1771 SR Wieringerwerf (NL); HOVELING, Richelle Johanna Maria, 1623 JA Hoorn (NL)
(74) Representative: Seemann & Partner Patentanwälte mbB

(57) **Abstract**

The present invention relates to a method and a system for providing a perfusion based complication prediction, a clinical decision support system, and a computer program.

The method comprises loading ICG perfusion image time series of a patient's organ receiving blood supply from the operated on blood vessel from times T1, T2 and T3 from a database, T1 denoting a time before clamping shut the blood vessel, T2 denoting a time after fully clamping shut the blood vessel and before repair of the blood vessel and T3 denoting a time after finishing the repair of the blood vessel and removing the clamp, normalizing the T1, T2 and T3 perfusion image time series to a common standard and extracting ICG fluorescence intensity time series for a plurality of regions of interest on an organ supplied by the vessel, taking a set of one or more collective characteristics of the fluorescence intensity time series at T1 and T2 as benchmarks for unobstructed and maximally obstructed blood supply, respectively, evaluating the same set of collective characteristics of the fluorescence intensity time series at T3 with respect to its collective proximity to those of the perfusion image time series at T1 and T2, respectively, the proximity to either being taken as an indicator for complication prediction, and outputting a complication prediction.

## Description

The present invention relates to a method and a system for providing a perfusion based complication prediction, a clinical decision support system, and a computer program.

Colonic ischemia (CI) is a complication that can occur after abdominal aortic aneurysm (AAA) repair surgery. This complication significantly affects the morbidity and mortality of this type of surgery. Often, this complication will only be detected days or weeks after the surgery has been performed. Earlier detection of CI can help reduce the morbidity and mortality of AAA repair surgery.

ICG (indocyanine green) fluorescence imaging is frequently used in evaluating perfusion of organic tissues. An example is colonic perfusion examined during abdominal aortic aneurysm (AAA) repair. During AAA repair, the aorta is clamped to prohibit blood flow through the aorta while the damaged aorta is repaired. After completion of the repair of the aorta, the clamp is removed and blood is once again allowed to flow freely through the aorta.

In the past, it has been known for the surgeon to evaluate colonic perfusion using ICG fluorescence by visual inspection or using non-standardized reference measurements, which does not yield reliable outcomes. One reason for this is that state-of-the-art methods for objective assessment of ICG fluorescence images often use generic cutoff values irrespective of the individual patients, making objective assessment unreliable, because fluorescence data can vary greatly between patients. This depends on multiple factors, including but not limited to camera settings, distance of the camera, such as an exoscope, to the surgical site and also on the patient himself. The latter in turn depends on how well the patient is able to adapt to impaired perfusion, e.g. by collateral perfusion.

In the light of this, it is an object of the present invention to provide means for objective assessment of the risk of complications due to perfusion issues in a patient.

This object is solved in an aspect of the present invention by a method for providing a perfusion based complication prediction for blood vessel related operation outcomes, the method in particular being carried out post-operatively or during an operation, wherein
a) ICG perfusion image time series of a patient's organ receiving blood supply from the operated on blood vessel from times T1, T2 and T3 are loaded from a database, T1 denoting a time before clamping shut the blood vessel, T2 denoting a time after fully clamping shut the blood vessel and before repair of the blood vessel and T3 denoting a time after finishing the repair of the blood vessel and removing the clamp,
b) the T1, T2 and T3 perfusion image time series are normalized to a common standard and ICG fluorescence intensity time series extracted for a plurality of Regions of Interest on an organ supplied by the vessel,
c) a set of one or more collective characteristics of the fluorescence intensity time series at T1 and T2 are taken as benchmarks for unobstructed and maximally obstructed blood supply, respectively,
d) the same set of collective characteristics of the fluorescence intensity time series at T3 is evaluated with respect to its collective proximity to those of the perfusion image time series at T1 and T2, respectively, the proximity to either being taken as an indicator for the complication prediction, and
e) a complication prediction based on the outcome of method step d) is output.

In particular, the method may not include surgical steps to be performed on the patient during an operation, in particular the surgery itself and the administration of ICG into the patient.

This evaluation forms the basis of an objective patient based perfusion assessment model for assessing colonic or other organic perfusion during or after surgery such as open abdominal aortic aneurysm (AAA) repair surgery. The model can aid the surgeon in making an informed decision about the patient's risk of developing complications such as colonic ischemia or in other cases where perfusion assessment is critical for patient outcomes.

The ICG fluorescence time series extracted for the plurality of Regions of Interest constitute normalized time intensity curves extracted from the ROIs or from the pixel or a combination of pixels from the ROIs.

In order to make the evaluation more robust, it is envisioned to additionally provide a region of interest on a different organic structure having less perfusion, such as the mesentery. Additionally or alternatively, it is also possible to use a piece of cloth to establish a baseline in the ICG fluorescence time series.

The ICG perfusion image time series may be open surgery near infrared (NIR) videos taken while ICG fluorescent marker is injected into the patient and show the spread and subsiding of ICG fluorescence in the perfused organs such as the colon. The time series may last for half a minute, a minute or two minutes.

After processing the time intensity curves, time intensity curve parameters can be calculated based on these curves. The number and selection of parameters can vary from patient to patient or from procedure to procedure. The selected parameters can include, but do not have to be limited to:
- average, maximum and/or variance of the ingress slope;
- average, maximum and/or variance in intensity during the ingress phase;
- average, maximum and/or variance of area under the curve (AUC) of ingress phase;
- average, maximum and/or variance of slope in the first 10 seconds after start of ingress;
- average, maximum and/or variance in intensity in the first 10 seconds after start of ingress;
- average, maximum and/or variance of area under the curve (AUC) of first 10 seconds after start of ingress; and
- average, maximum and/or variance of egress slope.

The terms average, median, maximum and variance refer to the collectivity of these parameters, since the curves of all regions of interest are evaluated collectively and their average, maximum or median values are used. The reason for the collective analysis of multiple ROIs is that no single ROI can be reliably identified and relied upon to yield dependable results and having dependable predictive value. On the other hand, since the analysis is a collective one, it is possible to define different regions of interest in the ICG image time series at T1, T2 and T3, as long as they are similarly distributed, which makes movements from one time series to the other less of an issue with respect to comparability.

In a further embodiment, the collective characteristics from the T1, T2 and T3 time series are scaled such that the value from T1 is positive, such as for example +1, the value from T2 is negative, such as for example -1, and the value from T3 is scaled accordingly. If multiple collective characteristics are used, a mean ratio of the T3 values may be calculated from the scaled T3 collective characteristics. A positive mean ratio will show a better resemblance of the T3 data to the T1 data and therefore stand for the preponderance of likelihood of a positive, complication free patient outcome, whereas a negative mean ratio will show a better resemblance of the T3 data to the T2 data and stand for the preponderance of likelihood of a negative patient outcome having complications. In other words, as the T1 video was taken before the AAA repair and the T2 video was taken after clamping the Aorta, the T1 video shows proper aortic blood supply to the colon and the T2 video shows impaired aortic blood supply to the colon. Therefore, if the T3 video shows a closer resemblance to the T2 video, this can be an indication of impaired aortic blood supply to the colon.

After placing the regions of interest, the ICG fluorescence intensity time series may be extracted by calculating the average NIR intensity for each region of interest for each frame. Once the ICG fluorescence intensity time series are extracted, the curves may be processed in further embodiments by subjecting the ICG perfusion image time series to image processing, wherein for each ICG perfusion image time series, the signal curves are filtered, normalized, and the baseline value is subtracted. The videos, i.e., the ICG perfusion image time series, may also be registered to remove most of the movement within the time series, or motion-tracking may be used on the ROIs to compensate for movements. This helps increase the quality of the extracted signal curves, i.e., the extracted ICG fluorescence intensity time series.

In further embodiments, the collective characteristics chosen for evaluation are determined beforehand based on T1 and T2 ICG fluorescence intensity time series extracted from ICG perfusion image time series of a plurality of patients and performing a statistical test on the extracted T1 and T2 ICG fluorescence intensity time series designed to identify those collective characteristics that show the most significant difference between their T1 and T2 values. The collective characteristics having the most distinctive difference between their T1 and T2 values are most indicative of impaired blood supply under clamping. This comparison may be done, e.g., using the Wilcoxon signed-rank test, a paired statistical test, or the t-test for matched pairs if the sample size is large enough.

In embodiments, at least one ingress-related parameter and at least one egress-related parameter may be selected for use in a decision model. Besides or in place of these parameters, the variance between the parameter values for each of the videos may also calculated. The variance of a parameter, in particular an ingress-related parameter, may be selected as another parameter for the decision model.

The object underlying the present invention is also solved by a computer-based Clinical Decision Support System (CDSS) capable of providing a perfusion based complication prediction for blood vessel related operation outcomes, the system comprising a computer connected to or comprising a database and running a software program configured to perform the above-described method.

With this, the CDSS embodies the same inventive ideas, characteristics, features and advantages as the method.

In embodiments, the CDSS further comprises a camera sensitive to the ICG fluorescence spectrum, an illumination device configured to provide excitation illumination for ICG, and a controller controlling the camera and the illumination device, wherein the camera is configured to capture ICG fluorescence image time series and the controller is configured to store the ICG fluorescence image time series in the database along with identifying information and information about the captured ICG fluorescence image time series being a T1, T2 or T3 time series.

In further embodiments, a method of operating on a patient may include capturing T1, T2 and T3 ICG perfusion image time series on a patient, T1 denoting a time before clamping shut the blood vessel, T2 denoting a time after fully clamping shut the blood vessel and before repair of the blood vessel and T3 denoting a time after finishing the repair of the blood vessel and removing the clamp, and analysing the T1, T2 and T3 ICG perfusion image time series according to the above-described method in order to arrive at a perfusion based prediction for blood vessel related operation outcomes.

In a further aspect, the object of the present disclosure is also solved by a computer program with software code that cause a computer of a computer-based CDSS (10), in particular as described above, to perform the steps of the above-described method.

Also envisioned is a method for surgery on a patient that involves clamping shut and repairing a blood vessel, in particular abdominal aortic aneurysm (AAA) repair surgery, comprising administering ICG to the patient at times T1, T2 and T3 and capturing ICG perfusion image time series of a patient's organ receiving blood supply from the operated on blood vessel at the times T1, T2 and T3, with T1 denoting a time before clamping shut the blood vessel, T2 denoting a time after fully clamping shut the blood vessel and before repair of the blood vessel and T3 denoting a time after finishing the repair of the blood vessel and removing the clamp, and carrying out a perfusion based complication prediction for blood vessel related operation outcomes according to the above-described method of the present disclosure.

The T1, T2 and T3 ICG perfusion image time series may be stored in a database for analysis.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic representation of a computer-based Clinical Decision Support System (CDSS),
- Fig. 2: a schematic representation of a method for providing a perfusion based complication prediction for blood vessel related operation outcomes,
- Fig. 3: a schematic representation of a method for choosing collective characteristics,
- Fig. 4: T1-, T2- and T3-fluorescence intensity time series of a first patient and a second patient,
- Fig. 5: outcomes of a selected characteristic at T1, T2 and T3 for a plurality of patients and
- Fig. 6: a table with outcomes of a perfusion based complication prediction.

In the drawings, the same or similar types of elements or respectively corresponding parts are provided with the same reference numbers in order to prevent the item from needing to be reintroduced.

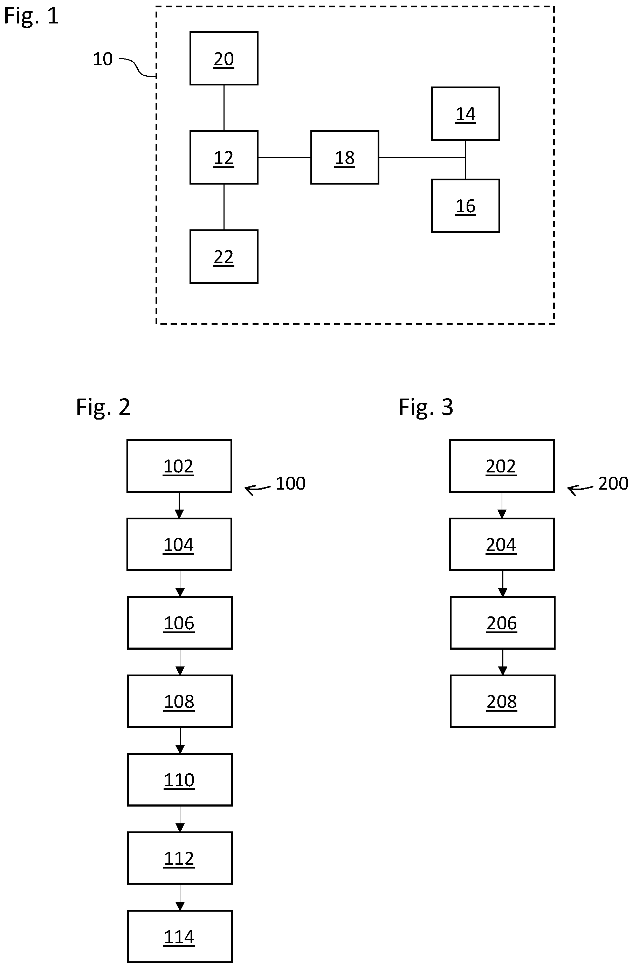

Fig. 1 illustrates a schematic representation of a computer-based Clinical Decision Support System (CDSS) 10 that is equipped and configured to carry out the inventive method of providing a perfusion based complication prediction for blood vessel related operation outcomes. The CDSS 10 has a central computer 12 running the software for the method. The computer 12 is connected to a controller 18 that controls a camera 14, such as an exoscope, and an illumination device 16 that is configured to provide illumination light for different illumination modes such as white light and infrared lighting for fluorescence excitation of the ICG fluorescence agent. The camera 14 is configured to capture image sequences, that is, videos, in the visible spectrum and in a fluorescence capture mode, particularly in the near infrared spectrum. The controller 18 is configured to control the capturing mode of the camera 14 and the illumination mode of the illumination device 16 to conform to white light imaging or fluorescence imaging, respectively, as the case may be.

The central computer 12 is in communication with the controller 18 and may be configured to instruct the controller 18 to perform control of the camera 14 of the illumination device 16 according to the image capturing mode required by the circumstances of a procedure. The computer 12 receives the captured image sequences from the camera 14 and stores them in a Database 20. Furthermore, the computer 12 is connected to a user interface device 22, such as a monitor, keyboard, mouse or the like.

Fig. 2 is a schematic representation of a method 100 for providing a perfusion based complication prediction for blood vessel related operation outcomes. It is assumed that, for example during an abdominal aortic aneurysm (AAA) repair surgery on a patient, fluorescence videos, i.e., ICG perfusion image time series have been taken by means of camera 14 and illumination device 16 of the CDSS 10, where an ICG fluorescence agent is injected during the operation at various times. During the operation, such ICG perfusion imaging time series are taken at a time T1 before clamping shut the aorta, then at a second time T2 after clamping shut the aorta, and then at a third time T3, which is after the AAA repair surgery has been completed and the clamping of the aorta has been removed. In each case, the videos show an organ whose blood supply comes mainly from the aorta, such as the colon. Other organs may, as the case may be, the ileum, the gastric conduit, trachea, the parathyroid gland among others.

In the case of AAA, the clamping shut of the aorta will have an effect on the perfusion of the colon with blood and hence with the ICG fluorescence agent. This agent is injected intravenously, usually the arm. However, in the aortal repair, patients often have central IV lines through the neck veins to the larger veins near the heart. Especially in the latter case, distribution of the ICG fluorescence agent can happen very quickly.

In step 102 of the method, the computer 12 loads the T1, T2, T3 ICG perfusion image time series from the Database 20 for further processing. A first processing step 104 may comprise normalizing the T1, T2, T3 ICG perfusion image time series. This step may also comprise filtering and subtracting of baseline values.

In step 106, multiple regions of interest are defined on the observed organ in the respective ICG perfusion imaging time series. Further regions of interest may be defined on control regions such as the mesentery, or a piece of cloth that does not have the ICG in it and therefore can serve as a baseline observation to react to changes in lighting. It is preferred that an equal number of regions of interest is defined in each of the T1, T2 and T3 ICG perfusion imaging time series, but this is not a strict requirement. The definition of multiple regions of interest helps in avoiding the results being defined by a single or just a few, possibly nonrepresentative, example or examples.

Also, the regions of interest need not be the same in all three ICG perfusion imaging time series, since from time T1 to time T2 to time T3 the patient or the camera may have been moved so that the organ under observation is located differently in all three videos and it is impossible or impractical to try to define the identical regions of interest over all three times. This impracticability is alleviated by the choice of multiple regions of interest in each of the three ICG perfusion imaging time series that provide statistical robustness to the analysis.

In step 108, T1, T2 and T3 ICG fluorescence intensity time series are extracted from the regions of interest that have been defined earlier. The normalization and baseline subtraction mentioned earlier may also or alternatively be applied to the ICG fluorescence intensity time series at this stage instead of earlier with respect to the ICG perfusion imaging time series. The difference is that, whereas the ICG perfusion imaging time series represent image series, that is, videos, the ICG fluorescence intensity time series represent only one value per region of interest per timeframe, namely the intensity of the fluorescence response.

The ICG fluorescence intensity time series of a single region of interest will therefore be a time dependent graph or curve that in many cases shows a steep rise during ingress of the ICG and a relatively flat decrease during the time that the ICG is metabolized through the liver, the so-called egress phase, whereas the ICG fluorescence intensity time series of all regions of interest combined will be a bundle of such time dependent curves that will be spread out somewhat, enabling a statistical analysis thereof including analysis of minimum, maximum, average values or their characteristic distributions such as variance, full width half maximum (FWHM) or the like. Normalization may mean that the maximum intensity value in any of the regions of interest is defined as, for example, 100%, with the scaling factor to this 100% value being taken as a normalizing factor and all the curves for all the regions of interest are normalized with that normalizing factor. For each of the T1, T2 and T3 ICG fluorescence intensity time series, the normalization may be done individually. However, if it can be ensured that lighting and positioning for the videos at the three different times did not change, the normalization may be performed with the same normalization factor for all three ICG fluorescence intensity time series. Furthermore, a baseline subtraction may also be performed with respect to the curves on the basis of the region of interest defined on a neutral part of the images, such as a piece of cloth or as a gray card placed in a non-obstructing part of the images.

In the next step 110, collective characteristics are extracted from the T1, T2, T3 ICG fluorescence intensity region of interest time series. This means, that the bundle of intensity curves are analyzed with respect to certain collective characteristics of the curve overall. Such characteristics may be determined for different phases of the curves, such as during ingress, up to maximum or a pre-defined time after start of ingress, such as 10 seconds, 20 seconds and so on, or during egress. Such characteristics may, e.g., be the slope, the value or the area under the curve during the various different phases. The collectiveness aspect may be realized by determining a maximum, average, minimum, variance or such of the characteristic under consideration for all the curves of the bundle from the multiple regions of interest. The analysis will usually be done for a number of such collective characteristics that have been shown to be most indicative of positive or negative patient outcomes.

In step 112, a proximity or proximities of extracted T3 collective characteristics to the respective extracted T1 and T2 collective characteristics is or are evaluated, where the T1 and T2 collective characteristic values represent the individual, i.e. subjective to the patient, ideal and worst case values. The T3 collective characteristic values will ideally resemble the T1 values for a positive outcome. If they resembled the T2 values more than the T1 values, this may indicate a possibly negative patient outcome. This is calculated in step 114 according to a weighting model and a complication prediction is output to the user.

Fig. 3 shows a schematic representation of a method 200 for choosing collective characteristics that can be used in method 100. The task at hand is basically defined those characteristics that, for a larger number of patients, provided the starkest distinction between the T1 and T2 states. For this purpose, in a first step 202, pre-normalized T1 and T2 ICG fluorescence intensity time series for multiple patients constituting a data pool are loaded from database 20. Alternatively, one or more of the steps 102, 104, 106, 108 and 110 of method 100 can be carried out for T1 and T2 ICG perfusion imaging time series from a sufficiently large number of patients undergoing the same or similar procedures, thus creating the plurality of normalized of T1 and T2 ICG fluorescence intensity time series for the patients from the data pool.

Next, a variety of collective characteristics is extracted from the T1 and T2 ICG fluorescence intensity time series in step 204 in a uniform fashion for all patients in the data pool, thus ensuring comparability and the ability to perform a statistical analysis in step 206 that is designed to determine those collective characteristics that are most significantly different for T1 and T2 fluorescence intensity time series, respectively. For example, in case of AAA repair surgery, the T2 ICG fluorescence intensity time series are compared to the T1 ICG fluorescence intensity time series for each patient in order to assess which parameters can be indicative of impaired aortic blood supply to the sigmoid colon, as the T2 videos all have impaired aortic blood supply to the sigmoid colon due to the clamping. This comparison may for example be done using the Wilcoxon signed-rank test, a paired statistical test. Since there may not be a direct relation between the plurality of regions of interest from the T1 and the T2 ICG perfusion imaging time series from the same patient, a single value for ICG perfusion imaging time series, i.e., video, will have to be calculated in order to perform the statistical test. To do this, the median value of each parameter from the plurality of regions of interest may be calculated for each video, resulting in a single value per parameter per video.

In step 208, the resulting collective characteristics definitions are output that provide the most reliable discrimination power between the T1 and the T2 characteristics.

In an exemplary embodiment, the statistical test results may be used to assess which parameters show a significant difference in values between T1 and T2. From those parameters, for example, one ingress-related parameter and one egress-related parameter may be selected for use in a decision model. Besides these parameters, the variance between the parameter values for each of the videos may also be calculated. The variance of an ingress-related parameter may for example be selected as the third parameter for the decision model. Other choices may be made as well.

Although the determination of the most distinguishing collective characteristics is done on a plurality of patient's ICG fluorescence intensity time series, the evaluation in method 100 of the individual risk of complications is done purely subjectively, that is, using only the individual patient's T1, T2 and T3 ICG fluorescence intensity time series data.

Referring again to Fig. 2, in step 112 of method 100, for the evaluation of the likelihood of complications of an individual patient, the value of that patient's T3 measurement would be compared to the values of the T2 and T1 measurements for that individual patient for each of the chosen parameter. It is then assessed whether the T3 measurement value is closer to the T2 measurement value or to the T1 measurement value, and by how much. The T3 value can be scaled based on the T2 and T1 values, to make the model quantitative and allow for better comparison between patients and parameters. Here, the T2 value may be defined as -1, and the T1 value may be defined as +1. The T3 value is then scaled accordingly. Afterwards, the mean value of the three T3 values (one for each parameter) can for example be taken to get a final value. Other weighting factors may be used based on the differing statistical significance of the various collective characteristics. Given the definition of the T1 and T2 values as +1 and - 1, if this value is negative, the T3 measurement more closely resembled the T2 measurement, indicating impaired aortic blood supply to the sigmoid colon. If this value is positive, the opposite is true.

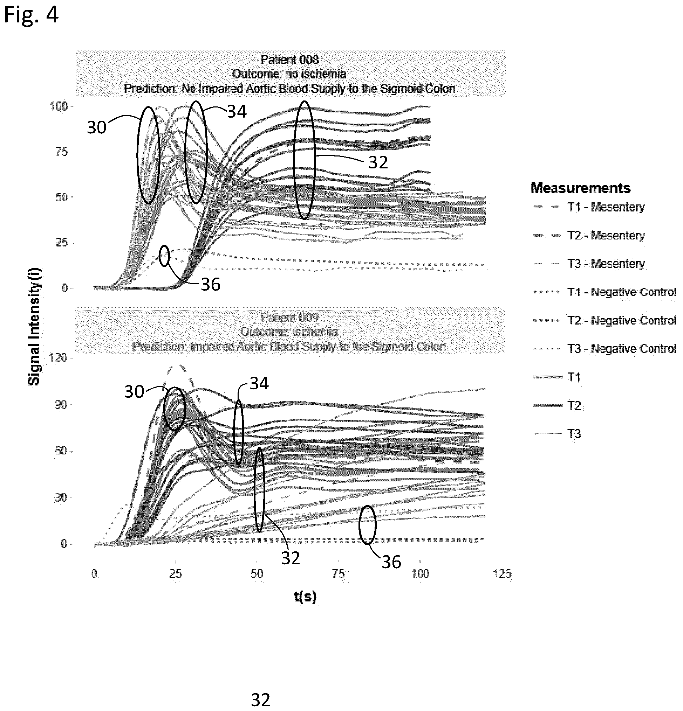

In Fig. 4, T1, T2 and T3 ICG fluorescence intensity time series derived from videos of the sigmoid colon during AAA repair surgery of a first patient and a second patient are shown as representative examples, with the data of the first patient in the upper half and the data of the second patient in the lower half of Fig. 4. In both cases, the T1, T2 and T3 ICG fluorescence intensity time series for all of the chosen regions of interest are displayed, with the horizontal axis representing a time axis spanning approximately two minutes from the start of the injection of the ICG fluorescence agent into the patient. The T1, T2 and T3 ICG fluorescence intensity time series are shown as bundles of curves that are individualized with reference number 30 in the case of the T1 series, reference number 32 in the case of the T2 series and reference number 34 in the case of the T3 series. The bundles of curves have been normalized such that, for each bundle, the maximum ICG fluorescence intensity is scaled to the value of 100. This also means, that only one of the curves of each bundle will reach the maximum value of 100.

Furthermore, a negative control is highlighted with reference number 36. Also, in each case there has been one region of interest been defined on the mesentery. The T1, T2 and T3 mesentery curves are shown in a dashed line. These usually lie within the group of curves for the regions of interest on the sigmoid colon. They have not been used in the statistical analysis and the comparison of the T3 values with the T1 and T2 values.

The ICG fluorescence intensity time series shown as curves in Fig. 4 have been subjected to some signal processing. These include filtering, wherein the curves were filtered using a Whittaker-Henderson filter with a lambda value of 500k. This filtering helped remove unwanted noise in the signal. Furthermore, a baseline subtraction was applied, wherein a baseline value was subtracted from each signal. The baseline value is defined as the average intensity of the curve before the average NIR value in the whole frame reaches >1% of its maximum intensity. Finally, the signals were normalized using a combined normalization method, wherein all signals of a video are scaled together so that the maximum value of the highest curve was at 100.

As can be seen in the upper half of Fig. 4, the T1 curves of the first patient start to rise early after the injection, reach a well defined maximum and starts to decrease to a lower level very quickly, where the lower level is around 50, that is, half of the maximum, and then decrease very slightly. The T2 curves show the impact of the clamping of the aorta. The rise during ingress of the ICG fluorescence agent starts later, is less steep and does not show a pronounced peak. Furthermore the spread, that is, the variance, of the curves of the T2 curves, is much larger than in the T1 case. The T3 curves resemble the T1 curves much more than the T2 curves in that they also rise quickly and present a pronounced maximum before going back to a reduced value of about 40% of the maximum. The spread is somewhat larger than in the T1 case. In this case, the decision model has predicted that the aortic blood supply to the sigmoid colon will not be impaired. The patient outcome was indeed such that no ischemia was observed.

In the lower half of Fig. 4, it can be seen that the T1 and T2 curves of the second patient differ from those of the first patient. The T1 curve indeed shows a rather quick rise during ingress and a maximum, wherein however the falloff is a little less pronounced than in the first patient. Also, the curves in the T1 bundle show a greater spread than in the case of the first patient. The T2 curves show a very different behavior. Most of the curves that are indicated by the ellipse with the reference number 32 rise very slowly, and they show a very large spread. Within the two minutes observation time, no peak can be observed and all of the curves are still rising. In the case of the T3 curves, there is once again a quick rise, but there is no pronounced maximum and the curves do not fall off significantly after the maximum is reached. In this case, although the initial rise has a steepness that is comparable to that of the T1 curves, the T3 collective characteristic values are actually closer to the T2 collective characteristic values so that the prediction is that the aortic blood supply to the sigmoid colon will be impaired. In this case, the patient suffered ischemia, proving the prediction correct.

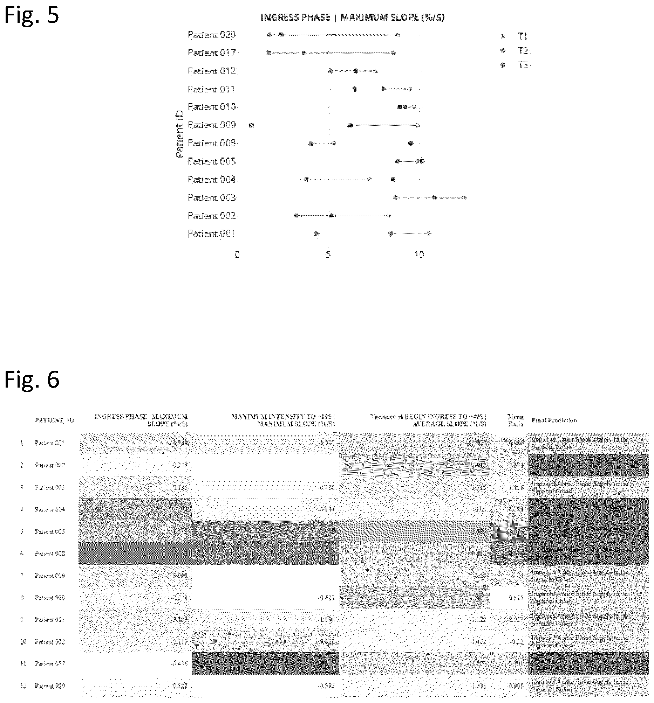

Fig. 5 illustrates outcomes of a selected characteristic at T1, T2 and T3 for a plurality of patients. The selected collective characteristic is the maximum slope during the ingress phase, that is the slope at its steepest point on the rising portion of the curves. For each patients in the data pool, the T1 and T2 values for this collective characteristic are displayed as endpoints of a line connecting the T1 and T2 values. In all cases, the T1 value is larger than the T2 value. In this case, no normalization to the range from -1 to +1 has been applied, the slope values are represented as percent per second (%/s). The respective T3 value is represented as a point that either lies on the line between the T1 and T2 values or outside the line, if the T3 value is smaller than the T2 value or larger than the T1 value.

In the case of patients 008 and 009, which are the same as those whose ICG fluorescence intensity time series curves are shown in Fig. 4, this particular collective characteristic lie above and, respectively, below the T1 and T2 values, giving patient 008 a good outlook and patient 009 a negative outlook with respect to possible complications. However, as can be seen, a single collective characteristic value may produce outliers, and the actual difference between the T1 and T2 values may be large in some cases and small in other cases, limiting their predictive value in individual cases. This is why it may be advisable to perform the analysis for several different collective characteristics and feed them into the decision model.

In Fig. 6, a table with outcomes of a perfusion based complication prediction is shown. In this case, the outcomes for three different collective characteristics are shown for the same data pool of patients, together with a mean ratio of the three characteristics and the final prediction for each patient. These collective characteristics comprise the maximum slope during the ingress phase that are shown in Fig. 5, the maximum slope in the time frame from maximum intensity to 10 seconds after the maximum intensity, and the variance of the average slopes in the time frame from beginning of the ingress to 40 seconds after the beginning of the ingress.

For example, in the case of patients 008 and 009, the first value is +7,736 and -3.901, respectively, which represent the fact that in Fig. 5, the T3 point in each case is a strong outlier with respect to the T1-T2 lines, one above, the other below. The values of +7,736 and - 3,901 for patients 008 and 009 are calculated as $value = \frac{T 3 - \left(T1+T2\right) / 2}{\left(T1-T2\right) / 2}$, which is the formula that defines the T1 value as +1 and the T2 value as -1. The same formula is used for the other values and patients as well.

In some cases, one of the three characteristic values could not be ascertained. In those instances, the mean ratio was calculated as the arithmetic middle of the remaining two values, in all other cases as the arithmetic middle of all three values. The final prediction of impaired or non-impaired aortic blood supply to the sigmoid colon depended on whether the mean ratio came out positive or negative.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 10: Clinical decision support system
- 12: computer
- 14: camera
- 16: illumination device
- 18: controller
- 20: database
- 22: user interface device
- 30: normalized T1 fluorescence signals
- 32: normalized T2 fluorescence signals
- 34: normalized T3 fluorescence signals
- 36: negative control signals
- 100: method
- 102: load T1, T2, T3 ICG perfusion image time series from database
- 104: normalize T1, T2, T3 ICG perfusion image time series
- 106: define regions of interest
- 108: extract T1, T2, T3 ICG fluorescence intensity time series from the regions of interest
- 110: extract collective characteristics from the T1, T2, T3 ICG fluorescence intensity region of interest time series
- 112: evaluate proximity of extracted T3 collective characteristics to extracted T1 and T2 collective characteristics
- 114: calculate and output a complication prediction
- 200: method for determining collective characteristics
- 202: load pre-normalized T1, T2 ICG fluorescence intensity time series for multiple patients from database
- 204: extract variety of collective characteristics from ICG fluorescence intensity time series

- 206: perform statistical analysis to determine those collective characteristics that are most significantly different for T1 and T2 fluorescence intensity time series, respectively
- 208: output resulting collective characteristics definitions

## Claims

1. A method for providing a perfusion based complication prediction for blood vessel related operation outcomes, the method in particular being carried out post-operatively or during an operation, wherein
a) ICG perfusion image time series of a patient's organ receiving blood supply from the operated on blood vessel from times T1, T2 and T3 are loaded from a database, T1 denoting a time before clamping shut the blood vessel, T2 denoting a time after fully clamping shut the blood vessel and before repair of the blood vessel and T3 denoting a time after finishing the repair of the blood vessel and removing the clamp,
b) the T1, T2 and T3 perfusion image time series are normalized to a common standard and ICG fluorescence intensity time series extracted for a plurality of Regions of Interest on an organ supplied by the vessel,
c) a set of one or more collective characteristics of the fluorescence intensity time series at T1 and T2 are taken as benchmarks for unobstructed and maximally obstructed blood supply, respectively,
d) the same set of collective characteristics of the fluorescence intensity time series at T3 is evaluated with respect to its collective proximity to those of the perfusion image time series at T1 and T2, respectively, the proximity to either being taken as an indicator for the complication prediction, and
e) a complication prediction based on the outcome of method step d) is output.

2. The method according to claim 1, wherein additionally a region of interest on a different organic structure having less perfusion is provided, such as the mesentery, and/or a piece of cloth is used to establish a baseline in the ICG fluorescence time series.

3. The method according to claim 1 or 2, wherein the set of collective characteristics of the fluorescence intensity time series comprises one or more of
- average, maximum and/or variance of the ingress slope;
- average, maximum and/or variance in intensity during the ingress phase;
- average, maximum and/or variance of area under the curve (AUC) of ingress phase;
- average, maximum and/or variance of slope in the first 10 seconds after start of ingress;
- average, maximum and/or variance in intensity in the first 10 seconds after start of ingress;
- average, maximum and/or variance of area under the curve (AUC) of first 10 seconds after start of ingress; and
- average, maximum and/or variance of egress slope.

4. The method according to one of claims 1 to 3, wherein the collective characteristics from the T1, T2 and T3 time series are scaled such that the value from T1 is positive, such as for example +1, the value from T2 is negative, such as for example -1, and the value from T3 is scaled accordingly, wherein in particular if multiple collective characteristics are used, a mean ratio of the T3 values is be calculated from the scaled T3 collective characteristics.

5. The method according to one of claims 1 to 4, wherein for each ICG perfusion image time series, the signal curves are filtered, normalized, and the baseline value is subtracted.

6. The method according to one of claims 1 to 5, wherein the collective characteristics chosen for evaluation are determined beforehand based on T1 and T2 ICG fluorescence intensity time series extracted from ICG perfusion image time series of a plurality of patients and performing a statistical test on the extracted T1 and T2 ICG fluorescence intensity time series designed to identify those collective characteristics that show the most significant difference between their T1 and T2 values.

7. The method according to claim 6, wherein at least one ingress-related parameter and at least one egress-related parameter is selected for use in a decision model.

8. A computer-based Clinical Decision Support System (CDSS) (10) capable of providing a perfusion based complication prediction for blood vessel related operation outcomes, the system (10) comprising a computer (12) connected to or comprising a database (20) and running a software program configured to perform the method according to one of claims 1 to 7.

9. The computer-based Clinical Decision Support System (CDSS) (10) of claim 8, further comprising an camera (14) sensitive in the ICG fluorescence spectrum, an illumination device (16) configured to provide excitation illumination for ICG, and a controller (18) controlling the camera (14) and the illumination device (16), wherein the camera (14) is configured to capture ICG fluorescence image time series and the controller (18) is configured to store the ICG fluorescence image time series in the database (20) along with identifying information and information about the captured ICG fluorescence image time series being a T1, T2 or T3 time series.

10. A computer program with software code that cause a computer (12) of a computer-based CDSS (10), in particular according to claim 8 or 9, to perform the steps of the method according to one of claims 1 to 7.
